# EUROPÄISCHE PATENTSCHRIFT

(11) **EP 3 092 175 B1**
(45) Veröffentlichungstag und Bekanntmachung des Hinweises auf die Patenterteilung: **27.12.2017**
(21) Anmeldenummer: 15700018.3
(22) Anmeldetag: 05.01.2015
(51) Int. Cl.: B65D 65/42, B65B 55/04, B65D 75/00, D21H 27/10, B65B 31/04, A61L 2/18, B65B 31/08, B65B 55/10, B65D 81/24, B65D 85/62, A61L 2/22

(54) **VERFAHREN ZUR BEHANDLUNG DER OFFENEN SCHNITTKANTEN VON VERPACKUNGSMATERIAL FÜR DIE FERTIGUNG VON KARTON/KUNSTSTOFFVERBUNDPACKUNGEN SOWIE VON VERPACKUNGSMATERIAL**
METHOD FOR TREATING THE OPEN CUT EDGES OF PACKAGING MATERIAL FOR THE PRODUCTION OF CARDBOARD/PLASTIC COMPOSITE PACKAGES OR A PACKAGING MATERIAL
PROCÉDÉ DE TRAITEMENT DES BORDS DE COUPE EXPOSÉS DE MATÉRIAU D'EMBALLAGE DESTINÉ À LA PRODUCTION D'EMBALLAGES COMPOSITES EN CARTON/PLASTIQUE AINSI QUE D'UN MATÉRIAU D'EMBALLAGE

(30) Priorität: 09.01.2014 DE 102014100203
(43) Veröffentlichungstag der Anmeldung: 16.11.2016
(60) Teilanmeldung: 17176407.9
(73) Patentinhaber: SIG Technology AG, 8212 Neuhausen am Rheinfall (CH)
(72) Erfinder: MAINZ, Hans Willi, 52525 Heinsberg (DE)
(74) Vertreter: Cohausz & Florack
(86) Internationale Anmeldenummer: PCT/EP2015/050028
(87) Internationale Veröffentlichungsnummer: WO 2015/104234

(56) Entgegenhaltungen:
- DE-A1- 19 748 022
- DE-B3-102011 111 523
- US-A- 5 900 111

## Beschreibung

Die Erfindung betrifft ein Verfahren zur Behandlung von offenen Schnittkanten eines Packungsmantels, Zuschnitts oder einer zu einer Rolle aufgerollten Bahnware eines Verpackungsmaterials, insbesondere Karton/Kunststoff-Verbundmaterials, durch Aufbringen bzw. Einbringen eines ein Entkeimungsmittel aufweisenden Behandlungsmittels auf bzw. in den äußeren Bereich der Schnittkanten.

Wenn im Folgenden von ,offenen Schnittkanten' die Rede ist, so sollen darunter nicht nur die Schnittkanten verstanden werden, die einen Zuschnitt oder einem daraus geformten Packungsmantel begrenzen, sondern sämtliche "offenen" also der umgebenden Atmosphäre zugänglichen Zellstoffbereiche, so dass insbesondere auch Schnittkanten im Inneren von Perforationen gemeint sind, wie sie bei Getränkepackungen im Bereich von aufzubringenden Ausgießelementen oder anderer Öffnungshilfen anzutreffen sind.

Die Herstellung von Karton/Kunststoff-Verbundpackungen erfolgt entweder als sog. "Schlauchformungsverfahren" von der Rolle oder aus einzelnen Zuschnitten aus Papier/Kunststoff Laminatmaterial. Hier werden zunächst aus einer Rolle Verbundmaterial einzelne Zuschnitte gewonnen und diese anschließend mit einer dichten Längsnaht versehen, die im Allgemeinen durch Faltung und Versiegeln des Verbundmaterials und ggf. durch zusätzliches Überkleben mit einem Dichtstreifen erzeugt wird.

Die weitere Verarbeitung derart hergestellter Packungsmäntel, also das einseitige Verschließen auf der Ober- oder Unterseite der späteren Verpackung, das Entkeimen, das Befüllen und erneute Verschließen erfolgt meist direkt in der Füllmaschine.

Hier erfolgen dann Becherformung, Reinigung und gegebenenfalls Desinfektion bevor das Füllgut eingefüllt und die Verbundverpackung verschlossen und endgültig ausgeformt wird. Desinfektion und Befüllung erfolgen bei einer Herstellung einer so genannten aseptischen Verbundverpackung in der aseptischen Zone einer Füllmaschine. Erfolgt die Becherformung vor der Desinfektion beziehungsweise der Sterilisation, kann die Becherformung auch außerhalb der Aseptikzone stattfinden. Derartige Verfahren sind unter anderem in der DE 32 35 476 A1 und der DE 10 2009 029 706 A1 beschrieben.

Unabhängig vom Herstellungsverfahren erfolgt das Verschließen der Packung in der Regel durch Zusammenpressen und Versiegeln der Packstoffkanten beispielsweise durch Ultraschall mittels einer Sonotrode und einem Amboss. Es sind auch andere Verfahren zum Verschließen der Packung bekannt, beispielsweise elektromagnetische Induktion oder die Baufschlagung mit Heißluft in Verbindung mit mechanischem Verpressen.

Es hat sich herausgestellt, dass insbesondere Staub für die Kontamination mit Keimen verantwortlich ist, so dass bei allen Schritten die Vermeidung von Staub im Vordergrund stehen sollte. Dies kann durch Staubabsaugung während der Packungsmantelfertigung und Verringerung der Standzeiten der verwendeten Schneidmesser erfolgen. Bedingt durch die Fasern im Zellstoff des verwendeten Kartons bleiben jedoch die offenen Schnittkanten stets die 'Problembereiche' bei der Packungsfertigung. Leistungsfähige Absauganlagen schaffen hier in den meisten Fällen zwar Abhilfe, belasten den Produktionsprozess jedoch durch hohe Energiekosten und Geräuschemissionen.

Unter einer aseptischen Verpackung ist eine Verpackung zu verstehen, in die ein Füllgut, insbesondere ein Lebensmittel, unter aseptischen Bedingungen gefüllt wird. Dazu verwendete Füllmaschinen umfassen eine Aseptikzone, eine Art Reinraum, in dem sterile, also keimfreie Bedingungen herrschen, zu deren Aufrechterhaltung der Raum meist bis auf wenige Öffnungen verschlossen oder weitgehend verschlossen ausgebildet ist. Die darin gebildete Reinraumatmosphäre steht zudem durch Einfuhr von Sterilluft unter Überdruck, so dass keine Keime von außen eindringen können. Das Verpackungsmaterial wird dann kontinuierlich oder diskontinuierlich durch die Aseptikzone transportiert, wobei es in aufeinanderfolgenden Schritten sterilisiert, getrocknet, in einem oder mehreren Schritten gefüllt und verschlossen wird. Pro Bearbeitungsschritt lassen heutige Maschinen nach Stand der Technik, etwa eine Maschine aus der 24er-Baureihe der Anmelderin, je nach Packungsformat eine Bearbeitungszeit einer einzelnen Sterilisations- oder Füllstation von etwa 0,6 Sekunden bis etwa 0,85 Sekunden zu.

Bei den Kanten von Packungsmänteln handelt es sich um offene Schnittkanten eines ansonsten wasserdichten und ggf. auch eine Sauerstoffbarriere aufweisenden Laminatmaterials. Daher besteht die Gefahr des Austausches von Keimen (Mikroorganismen und Sporen) in erster Linie im Bereich der Kanten, wodurch eine Erhöhung der Keimbelastung des Materials erfolgen und schließlich auch eine Kontamination des Produktes beim sich anschließenden Füllen der Verpackung bzw. Verschließen zur fertigen Packung nicht ausgeschlossen werden kann.

Das Verschließen eines einseitig offenen, gefüllten Behälters aus Verbundmaterial birgt das Risiko, dass, insbesondere beim Versiegeln mit Ultraschall, aus den offenen Schnittkanten Staub aus dem Verpackungsmaterial geschleudert werden kann und dieser sowohl den aseptischen Bereich der Füllmaschine als auch die offenen Verpackungen selbst verunreinigen kann.

Als vegetative Mikroorganismen werden durch Zellteilung vermehrungsfähige Einzeller verstanden, die geeignet sind sich im Füllgut (,Produkt') einer Packung zu vermehren und dabei die Eigenschaften des Füllgutes zu verändern. Weiter umfasst der Begriff auch die Überdauerungsformen der vermehrungsfähigen Einzeller, wie zum Beispiel deren Sporen.

Diese Sporen sind meist sehr resistent gegen Veränderungen der sie umgebenden Umweltbedingungen. Wenn Mikroorganismen keine Umgebung zum Stoffwechsel und/oder zur Fortpflanzung vorfinden, haben einige Mikroorganismen die Möglichkeit, in ein Sporenstadium überzugehen.

Genauer sollen unter dem Begriff Mikroorganismen im Sinne der vorliegenden Anmeldung Eukaryoten und Prokaryoten verstanden werden, wobei die Eukaryoten eine echte Zellwand aufweisen und Algen, Protozonen, Pilze und Schleimpilze umfassen, während die Prokaryoten die Gruppe der Bakterien abdecken (vgl. 'Bergey's Manual of Determinative Bacteriology', 8. Auflage, Baltimore: Williams & Wilkins, 1974).

Speziell bei den Prokaryoten sind Überdauerungsformen, wie beispielsweise Sporen, bekannt. Diese sind vermehrt beispielsweise auch nach thermischen und/oder chemischen Behandlungen von Rohstoffen zur Herstellung von Kartonroherzeugnissen in eben diesen anzutreffen, da derartige Behandlungsmethoden die direkt vermehrungsfähige Form der Mikroorganismen entweder abtötet oder den Übergang in die Sporenform initiiert.

Als Maß für die Anzahl beziehungsweise die Menge der in einer Stoffmenge, (beispielsweise in dem angesprochenen Kartonroherzeugnis) enthaltenen Mikroorganismen, ist dem Fachmann der Begriff der "koloniebildenden Einheit pro Gramm" (kbE/g) bekannt. Im Unterschied zu der direkten Auszählung aller vorhandenen Mikroorganismen mit einem geeigneten optischen Mittel, erfolgt die Bestimmung der Anzahl der koloniebildenden Einheiten über die gezielte Vermehrung der vorhandenen teilungsfähigen Mikroorganismen unter geeigneten Anzuchtbedingungen. Im Allgemeinen geschieht dies bis zu einer Koloniegröße, die mit dem unbewehrten Auge zählbar ist. Dabei nutzt man die Tatsache, dass aus jedem einzelnen teilungsfähigen Mikroorganismus unter zuvor definierten Bedingungen genau eine Kolonie entsteht. Einzelfälle, in denen zwei kbE so nah zusammenliegen, dass sich nur eine sichtbare Kolonie aus ihnen bildet, werden dann regelmäßig vernachlässigt.

In der Mikrobiologie typische Bestimmungsverfahren sind in der ISO 8784-1 aus 2005 geregelt.

Eine Reduzierung der kbE/g wird vom Fachmann demzufolge als Maß für die Wirksamkeit eines Verfahrens zur Keimreduktion verwendet und häufig Entkeimungsrate genannt. Daraus abgeleitet ergibt sich die über die Anzahl von produzierten Packungen zu zählende Sterilitätsrate.

Es ist aus der DE 10 2011111 523 A1 bereits bekannt, oben bzw. unten offene Schnittkanten eines Packungsmantels eines Verpackungsmaterials durch Aufbringen eines Behandlungsmittels, welches ein Entkeimungsmittel enthält, zu behandeln, wobei das Entkeimungsmittel nach dem Aufbringen auf den Schnittkanten verbleibt und in das Verpackungsmaterial eindringt. Das Aufbringen geschieht dabei jeweils durch Besprühen von oben, wobei eine Mehrzahl von Packungsmänteln flachgefaltet zusammengefasst sind. Bei diesem bekannten Verfahren erfolgt das Aufbringen des Behandlungsmittels auf die Packungsmantelkanten in einer eigens dafür konstruierten Station in einem oder mehreren separaten Verfahrensschritten unmittelbar vor dem Verpacken der Packungsmäntel in einen Umkarton. Diese Vorgehensweise ist relativ aufwändig. Auch besteht die Gefahr, dass versehentlich an der Packung vorbei gesprüht wird. Zudem hat sich gezeigt, dass die Außenseiten der Packungsmäntel rasch in Mitleidenschaft gezogen werden können. Beispielsweise kann das Behandlungsmittel in ungewollter Weise auf das Druckbild auf der Außenseite des Packungsmantels einwirken und dieses beschädigen. Auch kann es vorkommen, dass zwei Packungsmäntel nach Entnahme aus dem Umkarton aneinander kleben bleiben und bei ihrer weiteren Verarbeitung in der Füllmaschine zu Produktionsstörungen führen können.

Davon ausgehend liegt der Erfindung die Aufgabe zugrunde, eine besonders Ressourcen schonende Verbundverpackung und ein dazu nötiges Herstellungsverfahren bereit zu stellen.

Diese Aufgabe wird dadurch gelöst, dass wenigstens ein Teil des Behandlungsmittels nicht unmittelbar auf die Schnittkanten aufgebracht wird, sondern dass eine Beladung eines Behandlungsträgers erfolgt, welcher das Behandlungsmittel aufnimmt, so dass eine sich einstellende aktiv sterilisierende Atmosphäre anschließend auf die offenen Schnittkanten des Packungsmantels, Zuschnitts oder der Bahnware einwirkt und die gewünschte Sterilisation vornimmt, wobei die sich einstellende aktiv sterilisierende Atmosphäre durch ein Behältnis von der Umgebung separiert wird.

Dabei kann eine Sterilisation bis in eine ausreichende Tiefe in den Karton hinein erreicht werden, ohne dass der bekannte und bewährte Ablauf der Packungsfertigung beeinflusst werden soll. Die "ausreichende Tiefe" wird dabei durch das jeweils verwendete Herstellungsverfahren bestimmt. Erfolgt die Nahtbildung mittels Ultraschall, so muss eine Sterilisation bis etwa 2 bis 3 mm Tiefe erfolgen, um zuverlässig auszuschließen, dass beim Siegelvorgang kontaminierter Staub aus der offenen Kante geschleudert wird. Sollte die Naht mittels Mikrowellentechnologie geschlossen werden, reicht auch eine Tiefe von wenigen Zehntel Millimeter aus.

Bevorzugt erfolgt die Übertragung des Behandlungsmittels oder wenigstens eines Teils des Behandlungsmittels indirekt durch Verdampfung und anschließende Absorption in der/den offenen Schnittkante/n.

Eine weitere Lehre der Erfindung sieht vor, dass wenigstens Teile des Behältnisses, bevorzugt Teile der Innenseite, mit Behandlungsmittel benetzt werden.

Zweckmäßiger Weise ist eine Inkubationszeit für das Entkeimungsmittel vorzusehen. Diese liegt üblicher Weise im Bereich von einigen Minuten bis zu mehreren Stunden und reicht aus, die Desinfektion der Kanten über die Transportzeit der Zuschnitte, Packungsmäntel beziehungsweise der aufgerollten Bahnware zu einer Füllmaschine sicherzustellen. In der Regel verbleibt das Behandlungsmittel dauerhaft im Verpackungsmaterial. Die Kantenbereiche der Zuschnitte, Packungsmäntel bzw. der aufgerollten Bahnware bleiben dann über viele Tage bis hin zu einigen Monaten keimfrei.

Dadurch wird die Umwelt in besonderem Maße geschont, da der Herstellungsprozess der für Getränkekartons üblichen speziellen Kartonsorten in erheblichem Maß auf den Einsatz belastender Chemikalien angewiesen ist und zudem auch der Energiebedarf gegenüber einer standardmäßigen Kartonherstellung erhöht ist.

Wenigstens eine offene Schnittkante der jeweiligen Packungsmäntel ist dabei ausreichend von Entkeimungsmittel durchdrungen. Das bedeutet, dass die an den Schnittkanten angesiedelten und den wenigstens einen zweiten Teilbereich bildenden Randbereiche auf eine ausreichend niedrige Keimbelastung von maximal der Hälfte reduziert sind.

Die Einwirkzeit ist, jeweils bezogen auf eine bestimmte Kartonsorte und -grammatur, abhängig vom verwendeten Behandlungsmittel bzw. den enthaltenen Beigaben, der Dosierung, der erwarteten Entkeimungsrate und der Umgebungstemperatur.

Eine Absorption von Behandlungsmittel durch die Kartonkante ist möglich, da der für die herzustellenden Packungen eingesetzte Karton hydrophil ist, und im allgemeinen als Fertigprodukt eine relative Feuchte von etwa 5,5 % - 8,5 % aufweist, d.h. die Feuchte des Kartons liegt weit unterhalb der Sättigungsgrenze, die sich aus den herrschenden Umweltbedingungen ergibt und allgemein mit der Gleichgewichtsfeuchte zur Umgebung identisch ist.

Der Zusatz eines polaren organischen Lösungsmittels, das einen geringeren Dampfdruck als das Wirkmittel, hier also das Entkeimungsmittel, aufweist, wirkt dabei als Beschleuniger. So begünstigt der Zusatz an Ethanol zu beispielsweise einer Peroxid-Lösung die Absorptionswirkung der Schnittkante und erhöht die Penetration des Peroxids in den Karton. Dadurch wird ein tieferes Eindringen des Entkeimungsmittels in die Schnittkanten ermöglicht, so dass bei geeignetem Verhältnis von Entkeimungsmittel und Ethanol bereits nach wenigen Stunden ein Randbereich des zu behandelnden Verpackungsmaterials von bis zu mehreren Millimetern Tiefe sterilisiert sein kann.

Ethanol gehört zu der Gruppe der polaren organischen Lösungsmittel, die allgemein als Beschleuniger wirken, wenn ihr Dampfdruck geringer ist als der des Wirkmediums. Ist der Dampfdruck des beigemischten polaren organischen Lösungsmittels höher als der des Wirkmittels, wirkt das Lösungsmittel als Verzögerer. Ein Beispiel eines Lösungsmittels mit einem höheren Dampfdruck ist DOWANOL®, ein Markenprodukt der DOW Chemical Company. Allgemein kann man sagen, dass niedermolekulare polare organische Lösungsmittel einen niedrigen Dampfdruck aufweisen und höhermolekulare organische Lösungsmittel einen hohen Dampfdruck aufweisen.

Zusätzlich wird die Sterilisationswirkung von einigen Sterilisationsmitteln synergetisch verstärkt.

### Bestimmungsmethode:

Zur näheren Definition der in der Beschreibung genannten Merkmale wird folgende Bestimmungsmethode festgelegt:
*Bestimmung der koloniebildenden Einheiten je Gramm (kbE*/*g)*

Die europäische Standardmethode ISO 8784-1:2005 und die in dieser Prüfvorschrift angeführten Referenzen werden als Grundlage für die Bestimmung der koloniebildenen Einheiten je Gramm(kbE/g) gewählt. Die Prüfvorschriften werden hier für die Untersuchung der kbE/g-Belastung von Verpackungs- bzw. Verbundmaterial aus Karton, Kunststoff und teilweise Aluminium angewendet. Sollten in der Durchführung der Probennahme und Bestimmung der kbE/g des Verpackungsmaterials Abweichungen von den zitierten Prüfvorschriften notwendig sein, so werden sie im Folgenden erläutert.

### 1.1.1. Probennahme und Probenvorbereitung

Die entnommenen Verpackungsmaterialproben dürfen nicht mit den Händen angefasst werden. Die Aufbewahrung geschieht in sterilen Probenahmegefäßen, vorzugsweise sterilen Plastikbeuteln. Die zu untersuchenden Bereiche werden mit einer sterilen Schere in Stücke geschnitten.

"Zu untersuchende Bereich" sind:
a. Der erste Teilbereich, entsprechend Anspruch 10, der kein Randbereich ist (Probennahme min. 10 Millimeter von der offenen Schnittkante entfernt)
b. Der zweite Teilbereich bzw. Randbereich, entsprechend Anspruch 10, an den offenen Schnittkanten bzw. den Perforationskanten des Verpackungsmaterials (bis max. drei Millimeter ab der offenen Schnittkante)

In Ergänzung zur europäische Standardmethode ISO 8784-1:2005, Kapitel 8 "Preparation of the test material", werden für die ersten Teilbereiche max. 3 g Verpackungsmaterial eingesetzt. Für den zweiten Teilbereich werden ebenfalls max. 3 g Verpackungsmaterial eingesetzt. Sollte aus einem Probenstück nicht ausreichend Material erhalten werden, so werden die zweiten Teilbereiche aus max. 5 gleichen Probenstücken zusammen eingesetzt.

### 1.2. Optionale Bestimmung der Oberflächenkeimzahl

Es besteht die Möglichkeit, dass die Verpackungsmaterialproben vor der Probenahme zusätzlich zu dem Vorhandensein von Keimen im Innern auch oberflächlich verkeimt sind. Einen Fehler der Bestimmung der kbE/g des Verpackungsmaterials kann dadurch verhindert werden, dass die Oberflächenkeimzahl separat bestimmt wird und die Oberflächenkeimzahl von dem unter 1.1. bestimmten Zahlwert der koloniebildenden Einheiten je Gramm subtrahiert wird.

### 1.2.1. Begriffe und Abkürzungen

ml = Milliliter
h = Stunde
kbE = Kolonie bildende Einheiten
°C = Grad Celsius
g = Gramm
mm = Millimeter
cm = Zentimeter

### 1.2.2. Benötigte Hilfsmittel

Kontakt-Petrischalen (Kunststoff) ∅ 5,5 cm (z.B. Greiner Bio-one 629180) Pinzetten
Cutter-Messer
Steriler Folienbeutel oder Aluminiumfolie
Sterilisator
Brutschrank
sterile Werkbank
Nährmedien: Plate-Count-Agar (z.B. erhältlich als Oxoid Nr. CM 325, Merck Nr. 105463, Difco Nr. 247940)

### 1.2.3. Durchführung

Es werden pro Probe 240 cm² Verpackungsmaterialfläche untersucht.

Eine Kontakt-Petrischalen hat eine Fläche 24 cm². Daher sind 10 Kontaktschalen pro Probe vorzubereiten, um die o.g. Fläche zu untersuchen.

In die sterilen Petrischalen wird so viel Nährboden gegeben, dass der Agar über die Petrischalenkante hinausragt (Wölbung), jedoch nicht über den Rand hinausläuft. Die vorbereiteten erkalteten Kontaktschalen werden auf die Oberfläche des Verpackungsmaterials gedrückt, verschlossen und bei den angegebenen Bedingungen inkubiert.

Es ist darauf zu achten, dass die Probestücke nur mit einer sterilen Pinzette berührt werden und die Kontaktschalen nicht mit der offenen Packungskante in Berührung kommen.

Die Petrischalen werden mit dem Deckel nach unten in den Brutschrank gelegt, um Kondenswasserbildung zu vermeiden.

Nährboden: Für die Oberflächenkeimzahl wird Plate-Count-Agar verwendet.

Bebrütung:_Die Bebrütung des Plate-Count-Agars erfolgt 5 Tage bei 30 °C, wobei die Auswertung im Anschluss daran stattfindet.

### 1.2.4. Auswertung

Gezählt werden alle vorhandenen Kolonien auf den Kontaktplatten einer Probe. Die Ergebnisse werden auf kbE (koloniebildende Einheiten) / 100 cm² umgerechnet und dokumentiert. Der erhaltene Wert wird auf die Oberfläche der zu untersuchenden Bereiche umgerechnet und als Zahlenwert von der dem Wert der koloniebildenden Einheiten je Gramm subtrahiert.

### Beispiele:

Zur praktischen Umsetzung des erfindungsgemäßen Verfahrens kann als Behandlungsmittel beispielsweise eine Wasserstoffperoxidlösung von 3,5 % bis 50 % mit oder ohne Zusatz von
   - Verdampfungsverzögerer (beispielsweise DOWANOL®)
   - Mitteln die die Oberflächenspannung reduzieren, wie beispielsweise Alkoholanteile (Alkoholanteile mit Verzögerungsmittel, oberflächenaktive Substanzen wie Tenside etc.)
   - Peressigsäure 3-15 %
   - n- oder Isopropanol
   - anderen sporizid wirkende Mitteln
verwendet werden.

Die Lösung kann in Mischung mit Wasser oder Alkohol (Ethanol) verarbeitet werden, sie ist dabei ohne toxische Rückstände oder vollständig verflüchtigbar.
Selbstverständlich kann das Behandlungsmittel auch als Ersatz für die Wasserstoffperoxidlösung ein anderes geeignetes Entkeimungsmittel aufweisen.

In Tabelle 1 sind 12 Anwendungsbeispiele aufgeführt. Es gelten folgende Grundbedingungen für diese Beispiele:

Die Auswertung der kbE/g erfolgte nach der in den Methoden beschriebenen Normen und Verfahren. Das behandelte Verpackungsmaterial hat als Kartonlage einen Karton der Firma Stora Enso Natura Board mit einer Grammatur von 230 g/m². Wenn nicht anders beschrieben, wurden 350 Verpackungsmäntel in einem Behältnis aufbewahrt.

Die Behältnisse haben folgende Eigenschaften:
Umkarton: Aus Wellpappe hergestellter Container mit den Maßen B: 60 cm; H: 19 cm;
T: 10,5 cm
Kunststoffbox: Aus Polypropylen hergestellter Container mit Deckel mit den Maßen B: 60cm H: 19 cm T: 10,5 cm
Schrumpffolie: Aus Polyolefinen hergestellte Folie mit den Maßen B: 1m; L: 2,5 m; Dicke: 20µm
Beladener Papierträger: DIN A4, Zellstoff mit einer Grammatur von 150g/m²

Das Behandlungsmittel ergibt sich aus folgenden Chemikalien:
H₂O₂-Lösung: Peroxal DS 35% H₂O₂-Lösung (Lebensmittelqualität)
Ethanol: Ethanol technisch (99%) mit MEK vergällt (1%)
Methanol: Methanol technisch (98%)
Dowanol: 1-Methoxy-Propanol-2 von der Firma Dow

**Tabelle 1: Untersuchung verschiedener Anwendungsbeispiele**

| | Behandlungsmittel | Volumenanteil | Auftragsvolumen | Art der Auftragung | Behältnis | Unbehandelt * | 1 h Einwirkzeit* | 48 h Einwirkzeit* |
|---|---|---|---|---|---|---|---|---|
| Beispiel 1 | H2O2-Lösung | 1 | 1ml | Bestreichen der Umkartonlasche | Umkarton | -- | - | ++ |
| | Ethanol | 1 | | | | | | |
| | Wasser | 1 | | | | | | |
| Beispiel 2 | H2O2-Lösung | 10 | 10ml | Bestreichen der Umkartonlasche | Umkarton | -- | + | +++ |
| | Ethanol | 1 | | | | | | |
| | Wasser | 1 | | | | | | |
| Beispiel 3 | H2O2-Lösung | 1 | 5ml | Bestreichen der Umkartonlasche | Umkarton | -- | - | + |
| | Ethanol | 5 | | | | | | |
| | Wasser | 5 | | | | | | |
| Beispiel 4 | H2O2-Lösung | 2 | 2ml | Bestreichen der Umkartonlasche | Umkarton | -- | - | +++ |
| | Ethanol | 2 | | | | | | |
| | Wasser | 1 | | | | | | |
| Beispiel 5 | H2O2-Lösung | 2 | 5ml | Besprühen der Umkartonlasche | Umkarton | -- | + | +++ |
| | Ethanol | 2 | | | | | | |
| | Wasser | 1 | | | | | | |
| Beispiel 6 | H2O2-Lösung | 1 | 1ml | Besprühen der Umkartonlasche | Umkarton | -- | - | ++ |
| | Ethanol | 1 | | | | | | |
| | Wasser | 1 | | | | | | |
| Beispiel 7 | H2O2-Lösung | 2 | 1ml | beladener Papierträger | Kunststoffbox | -- | - | +++ |
| | Ethanol | 2 | | | | | | |
| | Wasser | 1 | | | | | | |
| Beispiel 8 | H2O2-Lösung | 2 | 2ml | beladener Papierträger | Umkarton | -- | - | ++ |
| | Ethanol | 2 | | | | | | |
| | Wasser | 1 | | | | | | |
| Beispiel 9 | H2O2-Lösung | 2 | 2ml | beladener Papierträger | Schrumpffolie | -- | - | +++ |
| | Ethanol | 2 | | | | | | |
| | Wasser | 1 | | | | | | |
| Beispiel 10 | H2O2-Lösung | 1 | 1 ml | Bestreichen der Umkartonlasche | Umkarton | -- | -- | - |
| | Ethanol | 1 | | | | | | |
| | Wasser | 5 | | | | | | |
| Beispiel 11 | H2O2-Lösung | 1 | 1ml | Bestreichen der Umkartonlasche | Umkarton | -- | - | ++ |
| | Methanol | 1 | | | | | | |
| | Wasser | 1 | | | | | | |
| Beispiel 12 | H2O2-Lösung | 1 | 1ml | Bestreichen der Umkartonlasche | Umkarton | -- | -- | - |
| | Dowanol | 1 | | | | | | |
| | Wasser | 1 | | | | | | |
| * KBEs im definierten Randbereich (bis zu 3 mm) der offenen Schnittkante des Verpackungsmaterials | | | | | | | | |

Legende:

| | | | |
|---|---|---|---|
| --- | > 1000 kbE/g | + | < 50 kbE/g |
| -- | 500 kbE/g < x < 1000 kbE/g | ++ | < 10 kbE/g |
| - | 100 kbE/g < x < 500 kbE/g | +++ | < 1 kbE/g |

Zusammenfassend können die Vorteile der Erfindung unter den folgenden Stichworten beschrieben werden:
- Behandlungsmittel kann definiert auf die Umkartonlaschen aufgebracht werden.
- Minimaler Verbrauch an Entkeimungsmittel im Vergleich zur Direktbesprühung der Kanten. Gefahrenpotenzial und Belastung für die Arbeitsumgebung sind dadurch deutlich verringert.
- Überbeladung durch direktes Aufsprühen wird vermieden.
- Zeit zum Absorptionsaustausch zwischen Kartonlasche/Mantelkante bzw. Perforation ist ausreichend vorhanden durch Transport vom Herstellort zum Abfüllbetrieb (häufig größer 3 Tage).
- Kennzeichnung durch Färben des Behandlungsmittels auf den Umkartonlaschen sinnvoll, daher gute Aufbringungs- und Dosierkontrolle möglich.
- Durch minimale Absorptionsmengen sind keine nachweisbaren Entkeimungsmittelrestmengen, z.B. Peroxidrestmengen vor Verarbeitung des Verpackungsmaterials an der Füllmaschine vorhanden. Eine Gesundheitsgefährdung ist daher ausgeschlossen.
- Keine Beeinflussung des sichtbaren Bereichs der Verpackungsaußenseite. Insbesondere wird das für den Verkauf der Verbundverpackung wichtige Druckbild nicht angegriffen und ein Aneinanderhaften benachbarter Packungsmäntel im Zuführprozess einer Füllmaschine wirksam verhindert.
- Das Behandlungsmittel, insbesondere die Peroxidlösung, wirkt praktisch ausschließlich auf die offenen Schnittkanten, weil es von diesen aktiv absorbiert wird. Keine Belastung der übrigen Packung.

Die Erfindung wird nachfolgend anhand einer lediglich Ausführungsbeispiele darstellenden Zeichnung näher erläutert. In der Zeichnung zeigen
- Fig. 1A: einen Zuschnitt für Getränkekartons in Draufsicht,
- Fig. 1B: einen flachgefalteten Packungsmantel, hergestellt aus einem Zuschnitt gemäß Fig. 1A,
- Fig. 2: einen Umkarton zur Aufnahme einer Vielzahl von Packungsmänteln mit oben offenen Kartonlaschen im Vertikalschnitt,
- Fig. 3: ein erstes Ausführungsbeispiel eines oben offenen Umkartons mit darin befindlichen flachgefalteten Packungsmänteln in perspektivischer Darstellung,
- Fig. 4: ein zweites Ausführungsbeispiel eines oben offenen Umkartons mit darin befindlichen flachgefalteten Packungsmänteln in perspektivischer Darstellung,
- Fig. 5: ein weiteres Ausführungsbeispiel eines oben offenen Umkartons mit darin befindlichen flachgefalteten Packungsmänteln in perspektivischer Darstellung,
- Fig. 6: ein anderes Ausführungsbeispiel eines Behältnisses im Vertikalschnitt,
- Fig. 7: ein Behältnis zur Aufnahme einer Rolle Verpackungsmaterial im Vertikalschnitt und
- Fig. 8: den Ausschnitt einer Ecke eines Behältnisses mit einem innen befestigten Trägerelement.

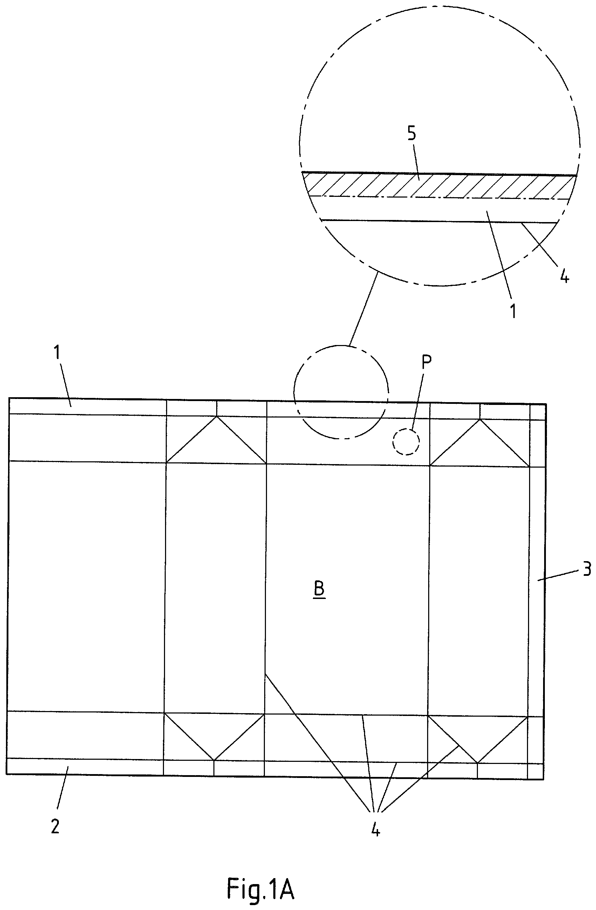

In Fig. 1A ist ein aus einem Verbundlaminat hergestellter Zuschnitt B dargestellt, der oben, unten und auf seiner rechten Seite drei Zonen 1, 2, 3 für die spätere Nahtherstellung aufweist und darüber hinaus mit einer Vielzahl von Rill- und Falzlinien 4 versehen ist, die das spätere Auffalten des fertigen Getränkekartons erleichtern und von denen nur einige in Fig. 1A mit dem Bezugszeichen 4 versehen sind. Auf der Oberseite des Zuschnitts B erkennt man eine runde Perforation P als Schwächungszone für ein aufzubringendes Ausgießelement (nicht dargestellt).

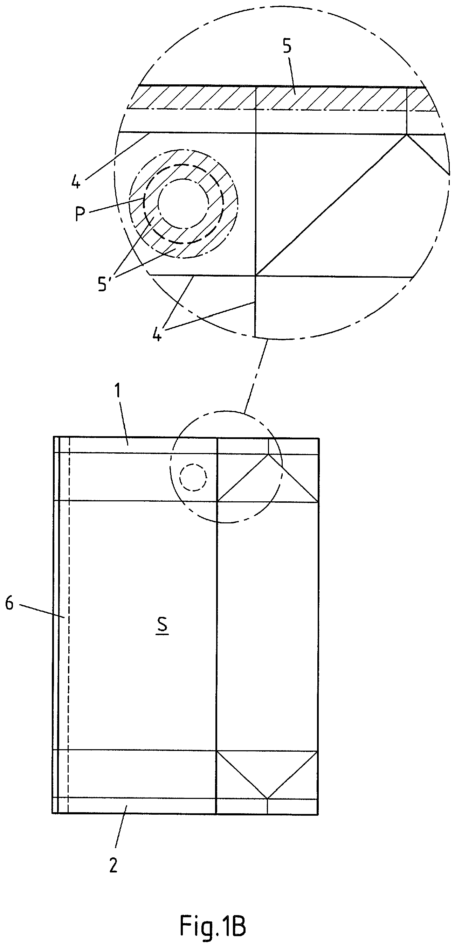

In Fig. 1B ist ein aus einem Zuschnitt B gemäß Fig. 1A hergestellter Packungsmantel S dargestellt, wobei der Zuschnitt B in seinem Nahtbereich 3 zu einer Hülse durch Siegelung einer Längsnaht 6 verbunden und zu einem Packungsmantel S flachgefaltet wurde. Das Flachfalten erfolgt aus einem einfachen Grund, da in der Regel die Herstellung der Packungsmäntel S an einem anderen Ort erfolgt als die spätere Weiterverarbeitung zu Getränkepackungen, welche in Abfüllbetrieben an unterschiedlichen Orten durchgeführt wird. Zu diesem Zweck wird eine Vielzahl flachgefalteter Packungsmäntel S aufrecht stehend in sog. Umkartons' 7 gepackt und zum jeweiligen Betrieb transportiert. Die Verweilzeit der Packungsmäntel S in den Umkartons 7 bis zu dessen Öffnung unmittelbar vor der Füllmaschine beträgt meist deutlich mehr als drei Tage.

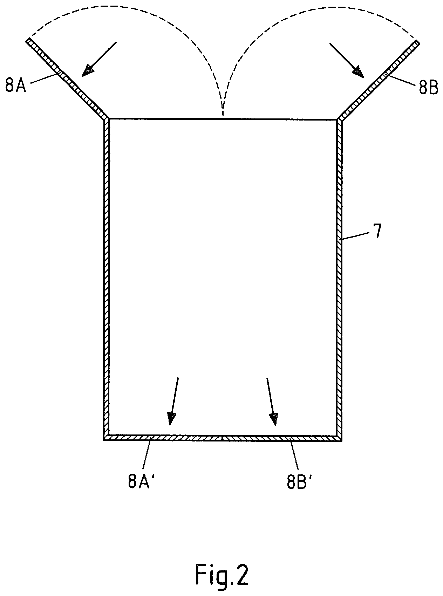

Die Erfindung hat nun erkannt, die hinsichtlich einer Kontamination problematischen offenen Packungskanten an der Unter- bzw. Oberseite der Packungsmäntel S nicht mehr direkt mit einem Entkeimungsmittel zu beaufschlagen, sondern derart, dass wenigstens ein Teil des das Entkeimungsmittel aufweisenden Behandlungsmittels auf ein Trägerelement aufgebracht wird, so dass die sich einstellende aktiv sterilisierende Atmosphäre anschließend auf die offenen Schnittkanten einwirkt und die gewünschte Sterilisation der Randbereiche 5 der Kanten bzw. der Ringbereiche 5' der Packungsmäntel S vornimmt. Als Trägerelement kann bevorzugt ein Umkarton 7 aus Wellpappe genutzt werden, bei dem die oberen und unteren Laschen 8A und 8B, 8A' und 8B' auf der Kartoninnenseite mit einem Behandlungsmittel benetzt werden und zwar im Bereich der nicht näher bezeichneten Pfeile in Fig. 2.

Auf diese Weise stellt sich im verschlossenen Umkarton 7 eine aktiv sterilisierende Atmosphäre ein, in der das Behandlungsmittel in die offenen Schnittkanten der Packungsmäntel S eindringen kann, beziehungsweise in der das im Randbereich oder einer Perforation an den offenen Schnittkanten angesiedelte Kartonmaterial dazu angeregt wird, das Behandlungsmittel zu absorbieren. Dort dringt das Entkeimungsmittel so weit (einige Millimeter) in das Kartonmaterial ein, so dass eine ausreichende Sterilisation erreicht wird.

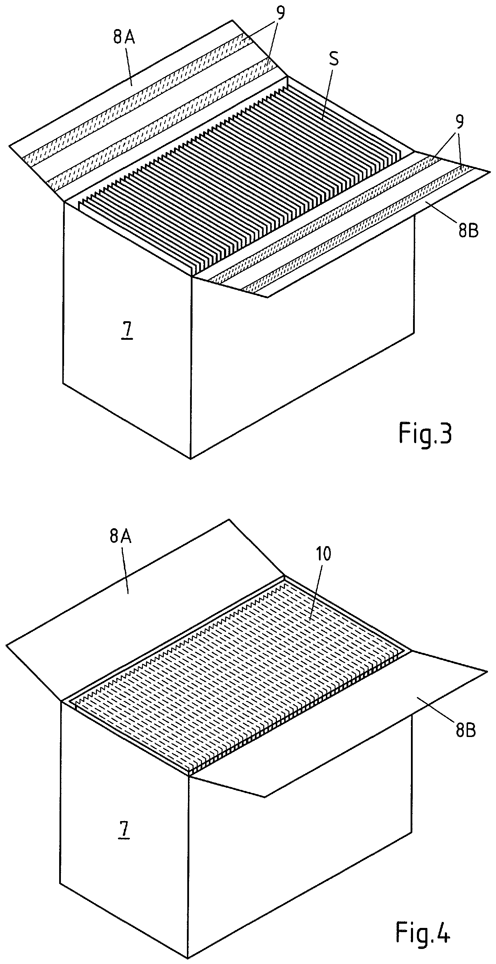

Es ist jedoch auch möglich, dass das Behandlungsmittel nicht direkt auf die Oberfläche des Umkartons 7 aufgebracht wird, sondern dort mit Hilfe eines Trägerelements oder Beladungsträgers eingebracht wird. Dazu können beispielsweise Streifen 9, wie in Fig. 3 dargestellt, aus Klebeband, Filz oder anderen geeigneten Materialien bestehen, welche auf die Innenseite des Umkartons 7 aufgeklebt werden oder im unteren Bereich des Umkartons 7 lediglich eingelegt werden können. Es hat sich gezeigt, dass eine vollflächige Benetzung nicht zwingend erforderlich ist, um eine ausreichende Sättigung der Atmosphäre mit Entkeimungsmittel zu erhalten.

Fig. 4 zeigt nun, dass es auch möglich ist, ein Einlegeblatt 10 in den Umkarton 7 (unten und/oder oben) einzulegen, wobei das Einlegeblatt 10 vollflächig oder auch nur teilweise mit der notwendigen Menge an Behandlungsmittel benetzt worden ist.

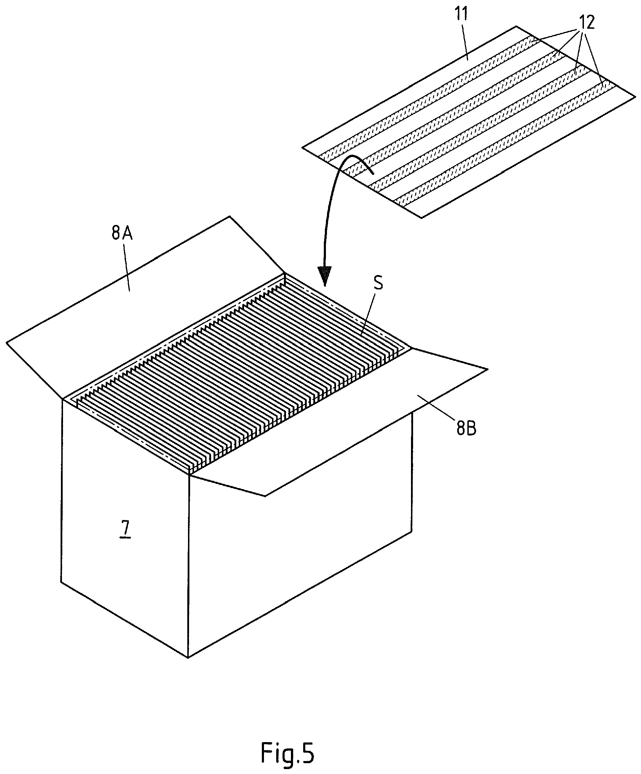

In Fig. 5 ist dargestellt, dass es auch möglich ist, ein Einlegeblatt 11 mit streifenförmigen Beladungsträgern 12 zu versehen, das dann anschließend umgekehrt auf die oberen offenen Schnittkanten der Packungsmäntel S aufgelegt wird, bevor der Umkarton 7 verschlossen wird. Selbstverständlich ist es auch möglich, vor dem Einbringen der Packungsmäntel S in den Umkarton 7 ein entsprechend vorbereitetes Einlegeblatt auf den Boden des Umkartons 7 zu legen, um auch die unteren offenen Schnittkanten der Packungsmäntel S zu entkeimen.

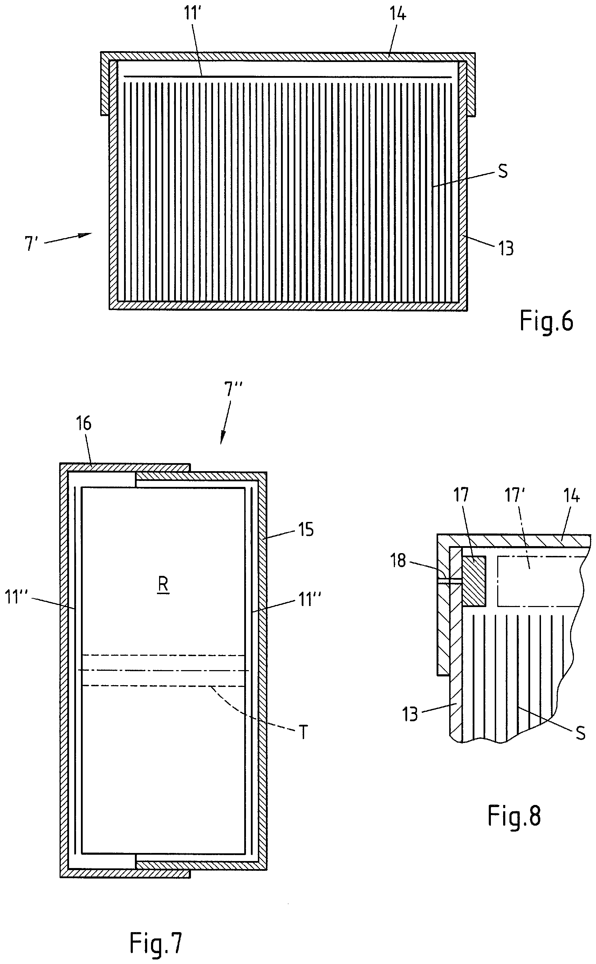

In Fig. 6 erkennt man, dass die aufrecht stehenden flachgefalteten Packungsmäntel S auch in einer ,Umverpackung' aus einem Kunststoffmaterial transportiert werden können. Man erkennt deutlich einen Kunststoffbehälter 13 im Vertikalschnitt auf dem ein entsprechender Deckel 14 aufgesetzt ist. In den Kopfraum dieser Umverpackung 7' ist ein Beladungsträger dienendes präpariertes Einlegeblatt 11' eingelegt, um die notwendige sterile Atmosphäre zu erreichen. Zum besseren Abschluss können die "offenen" Übergänge zwischen Behälter 13 und Deckel 14 auch noch mit einem Klebeband geschlossen werden.

Nicht dargestellt ist, dass es auch möglich ist, eine Mehrzahl von aufrecht stehenden und flachgefalteten Packungsmänteln nicht mit einer festen Umverpackung zu versehen, sondern beispielsweise mit einer Schrumpffolie zu umgeben, wobei vorher eine entsprechende Einlage aus Trägermaterial auf den Bereicht der offenen Schnittkanten gegeben wird, um dort die gewünschte Sterilisation durchzuführen.

In Fig. 7 ist dargestellt, dass es auch möglich ist, eine auf einer Hülse T aufgewickelte Rolle R eines Verpackungsverbundmaterials an seinen offenen Kanten mit dem erfindungsgemäßen Verfahren zu behandeln. Dies ist insbesondere dann von Vorteil, wenn die Rollenbreite genau der Packungshöhe entspricht, weil dann beide Kreisflächen der Rolle nahezu vollflächig aus offenen Schnittkanten bestehen. Dazu wird die Rolle R im dargestellten und insoweit bevorzugten Ausführungsbeispiel von einer zweiteiligen Schrumpfkappe bestehend aus einem kleineren Kappenteil 15 und einem daran angepassten größeren Kappenteil 16 umgeben. Man erkennt auch hier, dass im Bereich der offenen Schnittkanten Einlegeblätter 11" vorhanden sind, um die gewünschte sterile Atmosphäre zu schaffen. Selbstverständlich ist es auch bei der Verpackung von Rollenmaterial möglich, diese, wie zuvor beschrieben, nach dem Versehen mit Beladungsträgern 11" in Schrumpffolie zu verpacken.

In Fig. 8 ist nun am Bespiel der linken oberen Ecke des Containers aus Fig. 6 dargestellt, dass es auch möglich ist, als Beladungsträger einen etwas voluminöseren Streifen 17 aus Filzmaterial oder dergleichen einzubringen, wobei in diesem Fall eine Beladung des Streifens 17 mit Behandlungsmittel auch nach dem Verschließen des Behältnisses erfolgen kann. Dazu wird eine Wand des Behältnisses von einer Nadel durchstoßen und auf diese Weise der innenliegende Streifen 17 mit der notwendigen Menge an Behandlungsmittel getränkt, um die bereits fertig verpackten, flach gefalteten Packungsmäntel S an ihren offenen Schnittkanten ausreichend zu sterilisieren. Dazu kann der Container eine kanalvorbereitete Öffnung 18 aufweisen. Auch ist es möglich, falls notwendig, dass im Kopfraum des Containers weitere Streifen 17' als Beladungsträger vorgesehen sein können.

## Patentansprüche

1. Verfahren zur Behandlung von offenen Schnittkanten eines Packungsmantels, Zuschnitts oder einer zu einer Rolle aufgerollten Bahnware eines Verpackungsmaterials, insbesondere Karton/Kunststoff-Verbundmaterials, durch Aufbringen bzw. Einbringen eines ein Entkeimungsmittel aufweisenden Behandlungsmittels auf bzw. in den äußeren Bereich der Schnittkanten,
**dadurch gekennzeichnet, dass**
wenigstens ein Teil des Behandlungsmittels nicht unmittelbar auf die Schnittkanten aufgebracht wird, sondern dass eine Beladung eines Beladungsträgers erfolgt, welcher das Behandlungsmittel aufnimmt, so dass eine sich einstellende aktiv sterilisierende Atmosphäre anschließend auf die offenen Schnittkanten des Packungsmantels, Zuschnitts oder der Bahnware einwirkt und die gewünschte Sterilisation vornimmt, wobei die sich einstellende aktiv sterilisierende Atmosphäre durch ein Behältnis von der Umgebung separiert wird.

2. Verfahren nach Anspruch 1,
**dadurch gekennzeichnet, dass**
die Übertragung des Behandlungsmittels oder wenigstens eines Teils davon indirekt durch Verdampfung und anschließende Absorption in der/den offenen Schnittkante/n erfolgt.

3. Verfahren nach Anspruch 1,
**dadurch gekennzeichnet, dass**
als Beladungsträger wenigstens Teile des Behältnisses, insbesondere Teile einer Innenseite, mit Behandlungsmittel benetzt werden.

4. Verfahren nach Anspruch 1 oder 3,
**dadurch gekennzeichnet, dass**
als Beladungsträger in das Behältnis einzulegende oder einzuklebende Trägerelemente verwendet werden.

5. Verfahren nach einem der Ansprüche 1 bis 4,
**dadurch gekennzeichnet, dass**
die Beladung mit Behandlungsmittel vor dem Verschließen des Behältnisses erfolgt.

6. Verfahren nach einem der Ansprüche 1 bis 4,
**dadurch gekennzeichnet, dass**
die Beladung mit Behandlungsmittel nach dem Verschließen des Behältnisses erfolgt.

7. Verfahren nach einem der Ansprüche 1 bis 6,
**dadurch gekennzeichnet, dass**
ein Behandlungsmittel verwendet wird, das einen Farbstoff enthält und eine Verfärbung der behandelten Bereiche bewirkt.

## Claims

1. Method for processing open cut edges of a packaging cover, blank or web of a packaging material which has been rolled up into a roll, in particular cardboard/plastics composite material, by applying or introducing a processing agent which has a sterilisation agent onto or into the outer region of the cut edges,
**characterised in that**
at least a portion of the processing agent is not applied directly to the cut edges, but instead a loading of a loading carrier which receives the processing agent is carried out so that an actively sterilising atmosphere which is produced subsequently acts on the open cut edges of the packaging cover, blank or web and carries out the desired sterilisation, wherein the actively sterilising atmosphere which is produced is separated from the environment by a container.

2. Method according to Claim 1,
**characterised in that**
the transmission of the processing agent or at least a portion thereof is carried out indirectly by means of evaporation and subsequent absorption in the open cut edge(s).

3. Method according to Claim 1,
**characterised in that**
at least portions of the container, in particular portions of an inner side, are wetted with processing agent as a loading carrier.

4. Method according to Claim 1 or 3,
**characterised in that**
carrier elements which are intended to be laid or adhesively bonded in the container are used as the loading carrier.

5. Method according to any one of Claims 1 to 4,
**characterised in that**
the loading with processing agent is carried out before the closure of the container.

6. Method according to any one of Claims 1 to 4,
**characterised in that**
the loading with processing agent is carried out after the closure of the container.

7. Method according to any one of Claims 1 to 6,
**characterised in that**
a processing agent which contains a dye and which brings about discolouration of the processed regions is used.

## Revendications

1. Procédé de traitement de bords de coupe ouverts d'une enveloppe d'emballage, d'une découpe ou d'un produit - en bande continue - d'un matériau d'emballage, en particulier d'un matériau composite en carton / en matière plastique, ledit produit étant enroulé pour former un rouleau, ledit traitement étant effectué par application ou par introduction d'un moyen de traitement présentant un agent désinfectant, ledit moyen de traitement étant appliqué sur ou introduit dans la zone extérieure des bords de coupe,
**caractérisé**
**en ce qu'**au moins une partie du moyen de traitement n'est pas appliquée directement sur les bords de coupe, mais **en ce qu'**une charge d'un support de charge se produit, support de charge qui reçoit le moyen de traitement, de sorte qu'une atmosphère, qui se présente en ayant, de façon active, un effet stérilisant, agit ensuite sur les bords de coupe ouverts de l'enveloppe d'emballage, de la découpe ou du produit en bande continue, et effectue la stérilisation souhaitée, où l'atmosphère, qui se présente en ayant de façon active, un effet stérilisant à travers un récipient, est séparée du milieu environnant.

2. Procédé selon la revendication 1,
**caractérisé**
**en ce que** le transfert du moyen de traitement ou au moins d'une partie de celui-ci se produit indirectement par évaporation et par absorption qui se produit ensuite dans le ou dans les bord(s) de coupe ouvert(s).

3. Procédé selon la revendication 1,
**caractérisé**
**en ce que**, comme support de charge, au moins des parties du récipient, en particulier des parties d'un côté intérieur, sont mouillées avec un moyen de traitement.

4. Procédé selon la revendication 1 ou 3,
**caractérisé**
**en ce que**, comme support de charge, on utilise des éléments de support à insérer ou à coller dans le récipient.

5. Procédé selon l'une quelconque des revendications 1 à 4,
**caractérisé**
**en ce que** la charge avec un moyen de traitement se produit avant la fermeture du récipient.

6. Procédé selon l'une quelconque des revendications 1 à 4,
**caractérisé**
**en ce que** la charge avec un moyen de traitement se produit après la fermeture du récipient.

7. Procédé selon l'une quelconque des revendications 1 à 6,
**caractérisé**
**en ce qu'**un moyen de traitement est utilisé, moyen de traitement qui contient un colorant et déclenche une décoloration des zones traitées.
